Europäisches Patentamt

European Patent Office

Office européen des brevets

(11) Publication number: **0 126 453**
**B1**

(12) **EUROPEAN PATENT SPECIFICATION**

(45) Date of publication of patent specification: **27.12.90**

(51) Int. Cl.⁵: **A 61 K 9/22**

(21) Application number: **84105664.1**

(22) Date of filing: **18.05.84**

(54) **Controlled release tablet.**

(30) Priority: **19.05.83 US 496025**

(43) Date of publication of application:
**28.11.84 Bulletin 84/48**

(45) Publication of the grant of the patent:
**27.12.90 Bulletin 90/52**

(84) Designated Contracting States:
**AT BE CH DE FR GB IT LI LU NL SE**

(56) References cited:
**GB-A- 983 810**
**GB-A- 993 291**

(73) Proprietor: **MERRELL DOW PHARMACEUTICALS INC.**
**2110 East Galbraith Road**
**Cincinnati Ohio 45215-6300 (US)**

(72) Inventor: **Shah, Dhiren**
**8021 Lieber Road**
**Indianapolis Indiana 46260 (US)**
Inventor: **Gilbert, Deborah A.**
**7115 Antler Way, Apt. 2C**
**Indianapolis Indiana 46254 (US)**
Inventor: **Copeland, Robert D.**
**2711 Avon Road**
**Plainfield Indiana 46168 (US)**

(74) Representative: **Vossius & Partner**
**Siebertstrasse 4 P.O. Box 86 07 67**
**D-8000 München 86 (DE)**

Courier Press, Leamington Spa, England.

# EP 0 126 453 B1

**Description**

This invention relates to compression gradient pharmaceutical tablets which are useful in providing controlled release of the active ingredient.

Controlled relesae of medicaments from pharmaceutical dosage forms has long been considered desirable. Controlled action dosage forms are generally more convenient for the patient than are non controlled release dosage forms, requiring fewer interruptions of daily routine and nighttime sleeping habits. Moreover, controlled release dosage forms provide for a more extended release of active ingredient within the therapeutic range than do multiple doses of conventional dosage forms. Controlled release is typically achieved by a variety of techniques such as coated slow release beads, multiple layered tablets, tablets with slow release cores, and tablets employing porous inert carriers or ion exchange resins.

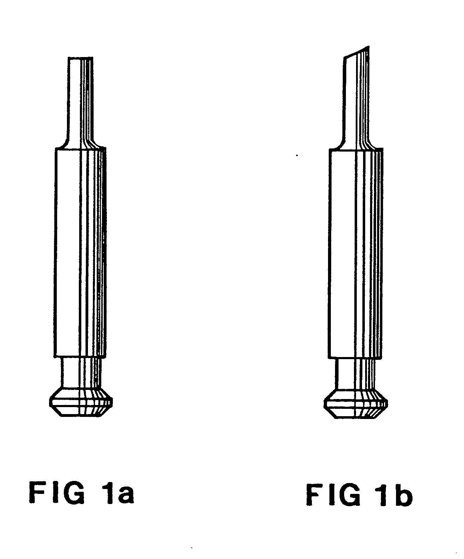

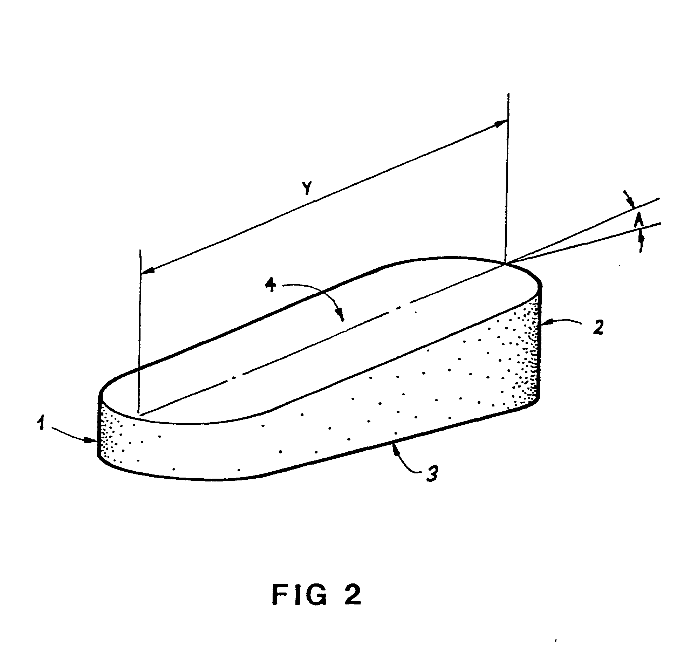

It has been discovered that a differentially compressed tablet provides a unique controlled release profile of the active ingredient. The differentially compressed tablets of this invention are prepared by modifying the normally horizontal faces of tablet press punches (see Figure 1a) so that either the upper or lower punch face or both the upper and lower punch faces are sloped (see Figure 1b). The sloped tablet punches produce tablets having sloped compressed faces as shown in Figures 2, 3 and 4. Tablets prepared in this manner will be differentially compressed, the thinner side being compressed with greater compressional force than the thicker side. The thinner side will be harder and will disintegrate and dissolve slower than the thicker side. Differentially compressed tablets dissolve slower and release active ingredients more slowly than do conventionally compressed tablets prepared from non-sloped tableting punch faces at comparable compressional force.

Figure 1 shows tableting punches:

(1a) a conventional tablet punch with a horizontal tableting face, and

(1b) a novel tablet punch with a sloped tableting face, the angle at which the punch face deviates from the horizontal is equal to the angle of inclination of a tablet produced therefrom,

Figure 2 is a view in perspective of a tablet structure of this invention having a capsule-shaped peripheral side, an upper sloped compressed face, a thinner side, 1, compressed at more force than the thicker side, 2, the length of the inclined or sloped face, Y, and the angle of inclination, A, which measures the declination of the sloped face from the horizontal plane, and a lower horizontal compressed face, 3,

Figure 3 is a view in perspective of a tablet structure of this invention having a capsule-shaped peripheral side, an upper sloped compressed face, a lower horizontal compressed face and beveled edges, and

Figure 4 is a view in perspective of a tablet structure of this invention having a capsule-shaped peripheral side, an upper sloped compressed face, a lower horizontal compressed face and rounded edges.

It has been discovered that a differentially compressed pharmaceutical tablet will provide for controlled release of the active ingredient. The differentially compressed tablets of this invention are prepared using conventional tableting techniques modified only in the use of a sloped or slanted tableting punch face in place of the normally horizontal punch faces. The thinner side of the tablets of this invention is harder and releases active ingredient more slowly than the thicker side which is softer. The compression gradient tablets of this invention provide for a more controlled release of the active ingredient than do conventional tablets.

Various factors influence the rate of release of active ingredient from the tablets of this invention including the active ingredient, the tablet excipients, the compression gradient and the shape of the peripheral side of the tablet. Drug substances which are readily soluble are expected to be released from a tablet of this invention more quickly than a drug substance which is only slowly soluble.

The pharmaceutical tablets of this invention can contain one or more drug substances, as well as various tablet excipients. Although any drug substances can be administered by controlled release, for various reasons, certain drug substances when administered via controlled release formulations, offer little or no advantage over more conventional modes of administration. For example, controlled release administration is contraindicated or is of questionable value for a) drugs with long biological half lives (i.e., greater than 10 hours) such as chlorpromazine and thioridazine; b) drugs absorbed by active transport, as for example, quaternary ammonium compounds such as propantheline bromide; c) antibiotics such as penicillins and cephalosporins; d) drugs destroyed by first pass liver metabolism and/or metabolism in the gut wall such as ritodrine, salicylamide or lidocaine; and drugs that are naturally sustained release, for example, medicaments which are absorbed in body fat and subsequently are slowly released into the patients blood, such as chlorpromazine and thioridazine.

Medicaments which are suitable for use in the tablets of this invention include any drug substance or combination of drug substances which can be formulated in a solid dosage form and for which controlled release administration would be desirable. Suitable drug substances and their indications are, for example, acetaminophen, an antipyretic; aminophylline, a smooth muscle relaxant; amitriptyline HCl, an antidepressant; betamethasone phosphate, a glucocorticoid; brompheniramine maleate, an antihistaminic; buphenine HCl, a peripheral vasodilator; carbetapentane citrate, an antitussive; carbochromene HCl, a coronary vasodilator; chlorpheniramine maleate an antihistaminic; chlorpromazine HCl, a tranquilizer; clonidine HCl; an antihypertensive; codeine phosphate, an antitussive; diethylpropion HCl, an anorexiant; dihydroergotamine mesylate, a cerebral vasodilator; dextromethorphan HBr, an

2

antitussive; diphylline, a bronchodilator; ergotamine tartrate, a vasoconstrictor; fenfluramine HCl, an anorexiant; ferritin, an antianemic; furosemide, a diuretic antihypertensive; heptaminol HCl, a cardiotonic; hydroquinidine HCl, an antiarryhthmic; ibuprofen, an anti-inflammatory; imipramine HCl, an antidepressant; indomethacin, an anti-inflammatory; isoxsuprine HCl, a vasodilator; isosorbide dinitrate, a coronary vasodilator; metformin HCl, a hypoglycemic agent; melperone, a neuroleptic; methscopalamine bromide, an antispasmodic; noscopine HCl, an antitussive; oxeladin citrate, an antitussive; papaverine HCl, a smooth muscle relaxant and cerebral vasodilator; pentazocine HCl, an analgesic; phenylephrine HCl, a sympathomimetic vasoconstrictor; phenylpropanolamine HCl, a sympathomimetic bronchodilator; potassium chloride, a potassium source for the treatment of hypokalemia; procainamide HCl, an antiarrhythmic; propranolol HCl, a beta receptor blocker, antihypertensive and antiarrhythmic agent; pseudoephedrine HCl, a bronchodilator and peripheral vasoconstrictor; theophylline, a smooth muscle relaxant, bronchodilator and myocardial stimulant; terbutaline sulfate, a bronchodilator; terfenadine, an antihistamine; and trihexyphenidyl HCl, an anti-Parkinsonian agent. Terbutaline, pseudophedrine HCl, terfenadine and melperone are preferred drug substances.

Any amount of medicament can be formulated into the tablets of the present invention. Due to size limitations, the tablets of this invention should not exceed about 500 mg of medicament. Applicants prefer dosage forms having 300 mg or less of medicament. In principle, there is no lower limit on the amount of medicament which can be formulated into the tablets of this invention. However, applicants prefer tablets having at least 0.1 mg of medicament. Preferred tablets of this invention will contain from 10 to 250 mg of active ingredient and will comprise from about 5 to 25 percent active ingredient by weight.

The tablets according to the invention can contain one or more excipients in addition to the active ingredient. Although any pharmaceutical tableting excipient can be used, it is desirable that the chosen excipients render the tablet disintegration rate, at least moderately, sensitive to changes in compressional force to provide for a significant controlled release effect. Suitable tablet excipients for use in applicants' tablets are, for example, diluents such as lactose, hydrogenated vegetable oils, dextrose, mannitol, sorbitol, gelatin, acacia, dicalcium phosphate, tricalcium phosphate or monocalcium phosphate; binders such as sucrose, polyethylene glycol, polyvinyl acetate phthalate (PVAP), hydroxypropylmethyl cellulose, polyvinylpyrrolidone; disintegrants such as starch, karoya gum, methylcellulose, ethylcellulose, sodium alginate or bentonite; lubricants such as stearic acid, zinc, calcium or magnesium stearate, or talc, colorants such as FD&C dyes and lakes, and flavoring agents (FD&C dyes: Food, drug and cosmetics dyes, approved by US Federal Drug Administration).

The compression gradient, the essential feature of the tablets of this invention, can be varied by changing the compressional force, the angle of inclination or the length of the sloped compressed face, Y, in Figure 2. Greater compressional force produces harder tablets which dissolve more slowly and provide a more controlled release of active ingredient. The compressional force used in the tablets of this invention can be any force sufficient to cause the tablet granulation to bind into a solid dosage form. Typically, tablets are compressed at from 150 to 3000 bar. In a preferred tableting process of the invention, the compressional force ranges from 500 to 1700 bar.

The angle of inclination of a tablet of this invention is the angle formed at the intersection of the sloped compressed face and a horizontal plane, angle A in Figure 2 and is a measurement of the declination of the sloped face from a plane perpendicular to the axis of compression.

The angle of inclination of applicants' tablets can be any angle greater than 0 degrees of arc up to about 30 degrees of arc, preferably from 5 to 25 degrees of arc. In a preferred embodiment of the present invention, the angle of inclination is 10 degrees of arc. In another preferred embodiment of the present invention, the angle of inclination is 25 degrees of arc.

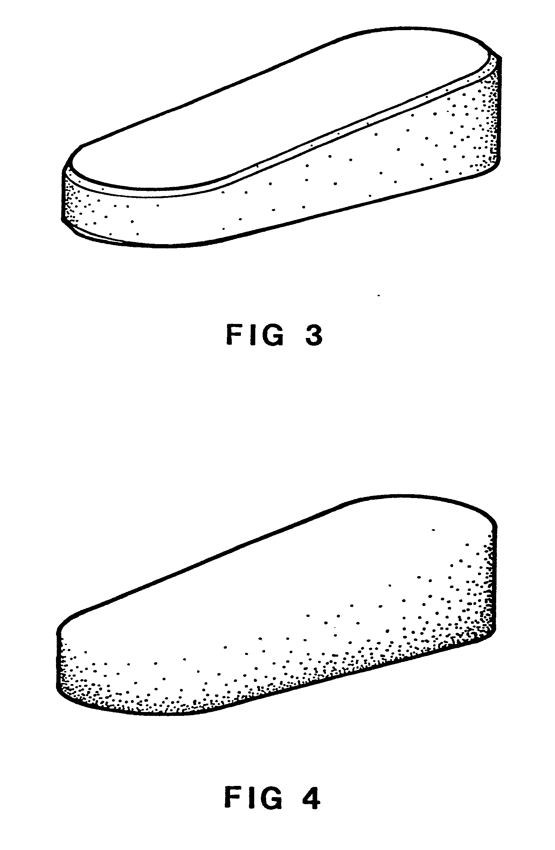

It should be kept in mind that the compressed faces and the peripheral sides of applicants' tablets need not be flat planar surfaces but may be convex, concave or otherwise rounded so long as one or both of the compressed faces are sloped. Where the tablet faces and sides are nonplanar, the angle of inclination can be determined by measurement of angle A, see Figure 2, along planes which average the rounded surfaces of the compressed faces and peripheral sides. It should also be noted that the surfaces of the tablets of this invention can be embossed with various designs and insignia without materially affecting the ability of these tablets to provide controlled release of active ingredient. Moreover, the edges of the tablets of this invention need not be sharply angular but may be beveled as in Figure 3 or rounded as in Figure 4.

The peripheral sides of the tablets of the present invention can be any shape including, rod, capsule, oval, ovoid, circular, square, triangular and trianguloid. Because it is desirable that the length of the sloped compressed face, length Y in Figure 2, be as long as possible in order to maximize the difference in compressional force from the thin tablet side, 1 in Figure 2, to the thick side of the tablet, 2 in Figure 2, certain peripheral side shapes are preferred. Among the preferred shapes are rod- and capsule-shaped sides. These shapes are preferred because the length of the sloped tablet face, Y in Figure 2, and the resulting compression gradient, can be maximized for a given tablet weight. Typical capsule-shaped compression gradient tablets, having length Y maximized by sloping along the longer capsule-shape axis, are illustrated in Figures 2, 3 and 4. Preferably in a capsule shaped tablet of this invention, the length of the sloped compression face, Y in Figure 2, would be from 5 to 20 mm in length.

The compression gradient tablets of this invention are prepared in a manner analogous to procedures readily known by those skilled in the art for preparing conventional compressed tablets but using modified tableting punches. The tablet punches used in preparing the compression gradient tablets of this invention are sloped or slanted as shown in Figure 1b. The declination of the sloped tablet punch is equal to the angle of inclination of the resulting compression gradient tablet. Either the upper or lower tableting punches or both the upper and lower tableting punches can be so modified. Additionally, the sloped tableting punch face may be modified to provide for convex, concave, or rounded edges or embossed designs and insignia. Tableting is performed using conventional tableting machines modified only by the use of the sloped face tableting punch.

The following examples illustrate the effect of a differentially compressed tablet on the release of active ingredient from a tablet dosage form.

Example 1

Prepare conventional and compression gradient tablets of 500 mg each at 439.4 bar compressional force having the following composition:

| | |
|---|---|
| d-Pseudoephedrine HCl | 12% |
| Methocel E4M® (a hydroxypropyl methyl-cellulose sold by the Dow Chemical company) | 30% |
| Methocel K4M® (a hydroxypropyl methyl-cellulose sold by the Dow Chemical Company) | 30% |
| Lubritab® (hydrogenated vegetable oil sold by Edward Mendell Company) | 12.5% |
| Lactose NF | 15% |
| Magnesium stearate | 0.5% |

Using a conventional tablet and compression gradient tablets having an angle of inclination equal to 10 degrees and 25 degrees of arc, a dissolution test was performed using the United States Pharmacopeia Method I involving 0.1 N hydrochloric acid at 37°C and stirred at 50 rpm. The $T_{50\%}$ and $T_{90\%}$ values indicate the controlled release effect of the compression gradient tablets and indicate that the compression gradient tablets having a larger angle of inclination produce a longer controlled release effect than the compression gradient tablet having a smaller angle of inclination. $T_{50\%}$ is the time required to release 50% of the active ingredient from the tablet. $T_{90\%}$ is the time required to release 90% of the active ingredient from the tablet.

| | Conventional tablet (min) | Compression gradient tablets angle of inclination | |
|---|---|---|---|
| | | 10° (min) | 25° (min) |
| $T_{50\%}$ | 214 | 122 | 152 |
| $T_{90\%}$ | 484 | 482 | 482 |

Example 2

Prepare conventional and compression gradient tablets weighing 600 mg each at 439.4 bar (6250 p.s.i.) compressional force having the following composition:

| | |
|---|---|
| d-Pseudoephedrine HCl | 10.1% |
| Lactose (hydrous) | 10.1% |
| Ethocel® (a solution of ethylcellulose in alcohol @ 22 cp. sold by the Dow Chemical Company) | .67% |
| Emcompress® (dicalcium phosphate sold by Edward Mendell Company) | 77.1% |
| Sta Rx1500 Starch | 1% |
| Magnesium stearate | 1% |

**EP 0 126 453 B1**

Using the dissolution test of Example 1 the following results were obtained:

| | | Conventional tablet (min) | Compression gradient tablets angle of inclination | |
| | | | 10° (min) | 25° (min) |
|---|---|---|---|---|
| $T_{50\%}$ | | 47.9 | — | 63.5 |
| $T_{90\%}$ | | 152.0 | — | 363.0 |

Example 3

Prepare conventional and compression gradient tablets weighing 600 mg each at 439.4 bar compressional force having the following composition:

| | |
|---|---|
| d-Pseudoephedrine HCl | 10% |
| Methocel® K100M (hydroxypropyl methyl-cellulose sold by The Dow Chemical Company) | 10% |
| Emcompress® (dicalcium phosphate sold by Edward Mendell Company) | 79% |
| Magnesium stearate | 1% |

Using the dissolution test of Example 1, the following results were obtained:

| | | Conventional tablet (min) | Compression gradient tablets angle of inclination | |
| | | | 10° (min) | 25° (min) |
|---|---|---|---|---|
| $T_{50\%}$ | | 91.7 | — | 105 |
| $T_{90\%}$ | | 391.7 | — | 480 |

Example 4

Conventional and compression gradient tablets having the composition set forth in Example 2 above were compressed at various pressures. The table below indicates the thickness of the tablets.

| Compression pressure (bar) | Regular tablet | Thickness (mm) Compression gradient tablet | |
| | | Thick side | Thin side |
|---|---|---|---|
| 186 | 5.62 | 5.91 | 4.78 |
| 582 | 5.09 | 5.43 | 4.32 |
| 1246 | 4.80 | 5.16 | 4.01 |
| 1676 | 4.76 | 5.05 | 3.90 |

**Claims**

1. A controlled release pharmaceutical tablet comprising compressed faces wherein at least one of the compressed faces is sloped characterized by a compression gradient which increases towards the thinner end of the tablet.

2. A tablet of Claim 1 wherein the angle of inclination is from 5 to 25 degrees of arc.

3. A tablet of Claim 1 wherein the angle of inclination is 25 degrees of arc.

4. A tablet of Claim 1 wherein the angle of inclination is 10 degrees of arc.

5. A tablet of Claim 1 wherein the peripheral side is capsule- or rod-shaped.

6. A process for preparing a pharmaceutical tablet providing for controlled release of the active ingredient, characterized by a compression gradient which increases towards the thinner end of the tablet by employing a tableting punch with a sloped face.

5

7. A process of Claim 6 wherein the angle of inclination of the sloped tableting punch is from 5 to 25 degrees of arc.

8. A process of Claim 6 wherein the angle of inclination of the sloped tableting punch is 10 degrees of arc.

9. A process of Claim 6 wherein the angle of inclination of the sloped tableting punch is 25 degrees of arc.

10. A process of Claim 6 wherein the resulting tablet has a capule- or rod-shaped peripheral side.

11. A tableting punch as used in any of Claims 6 to 10, characterized in that the tablet punch has a sloped tableting face.

12. A tableting punch of Claim 11, wherein the angle of inclination of the sloped tableting face is from 5 to 25 degress of arc.

13. The use of a tablet comprising compressed faces, at least one of which is sloped and which is characterized by a compression gradient which increases towards the thinner end of the tablet, for the controlled release of the active ingredient.

14. The use according to Claim 13, wherein the angle of inclination of the sloped faces is from 5 to 25 degrees of arc.

15. The use according to Claim 13, wherein the angle of inclination of the sloped faces is 25 degrees of arc.

16. The use according to Claim 13, wherein the angle of inclination of the sloped faces is 10 degrees of arc.

17. The use according to Claim 13, wherein the peripheral side of the tablet is capsule- or rod-shaped.

## Patentansprüche

1. Arzneimitteltablette mit gesteuerter Freigabe mit gepreßten Flächen, wobei mindestens eine der gepreßten Flächen geneigt ist, gekennzeichnet durch einen Kompressionsgradienten, der in Richtung des dünneren Endes der Tablette zunimmt.

2. Tablette nach Anspruch 1, wobei der Neigungswinkel zwischen 5 bis 25 Bogengrad beträgt.

3. Tablette nach Anspruch 1, wobei der Neigungswinkel 25 Bogengrad beträgt.

4. Tablette nach Anspruch 1, wobei der Neigungswinkel 10 Bogengrad beträgt.

5. Tablette nach Anspruch 1, wobei die Umfangsseite kapsel oder stabförmig ist.

6. Verfahren zur Herstellung einer Arzneimitteltablette, die eine kontrollierte Freigabe des Wirkstoffs bereitstellt, gekennzeichnet durch einen Kompressionsgradienten, der in Richtung des dünneren Endes der Tablette größer wird, durch Verwenden eines Tablettierstempels mit einer geneigten Fläche.

7. Verfahren nach Anspruch 6, wobei der Niegungswinkel des geneigten Tablettierstempels 5 bis 25 Bogengrad beträgt.

8. Verfahren nach Anspruch 6, wobei der Neigungswinkel des geneigten Tablettierstempels 10 Bogengrad beträgt.

9. Verfahren nach Anspruch 6, wobei der Neigungswinkel des geneigten Tablettierstempels 25 Bogengrad beträgt.

10. Verfahren nach Anspruch 6, wobei die resultierende Tablette eine kapsel- oder stabförmige Umfangsseite hat.

11. Tablettierstempel zur Verwendung in einem der Ansprüche 6 bis 10, dadurch gekenneichnet, daß der Tablettierstempel eine geneigte Tablettierfläche aufweist.

12. Tablettierstempel nach Anspruch 11, wobei der Neigungswinkel der geneigten Tablettierfläche 5 bis 25 Bogengrad beträgt.

13. Verfahren einer Tablette mit gepreßten Flächen, wobei mindestens eine geneigt ist und die gekennzeichnet ist durch einen Kompressionsgradienten, der in Richtung des dünneren Endes der Tablette zunimmt, zur gesteuerten Freigabe des Wirkstoffs

14. Verwendung nach Anspruch 13, wobei der Neigungswinkel der geneigten Flächen 5 bis 25 Bogengrad beträgt.

15. Verwendung nach Anspruch 13, wobei der Neigungswinkel der geneigten Flächen 25 Bogengrad beträgt.

16. Verwendung nach Anspruch 13, wobei der Neigungswinkel der geneigten Flächen 10 Bogengrad beträgt.

17. Verwendung nach Anspruch 13, wobei die Umfangsseite der Tablette kapsel- oder stabförmig ist.

## Revendications

1. Comprimé pharmaceutique à libération contrôlée comprenant des faces comprimées où au moins une des faces comprimées est inclinée, caractérisé par un gradient de compression qui s'accroît vers l'extrémité plus mince du comprimé.

2. Comprimé selon la revendication 1, dans lequel l'angle d'inclinaison est de 5 à 25° d'arc.

3. Comprimé selon la revendication 1, dans lequel l'angle d'inclinaison est de 25° d'arc.

4. Comprimé selon la revendication 1, dans lequel l'angle d'inclinaison est de 10° d'arc.

5. Comprimé selon la revendication 1 dont le côté périphérique est en forme de capsule ou de tige.

6. Procédé pour préparer un comprimé pharmaceutique assurant la libération contrôlée de l'ingrédient actif, caractérisé par un gradient de compression qui s'accroît vers l'extrémité plus mince du comprimé, par emploi d'un poinçon de compression à face inclinée.

7. Procédé selon la revendication 6, dans lequel l'angle d'inclinaison du poinçon de compression inclinée est de 5 à 25° d'arc.

8. Procédé selon la revendication 6, dans lequel l'angle d'inclinaison du poinçon de compression incliné est de 10° d'arc.

9. Procédé selon la revendication 6, dans lequel l'angle d'inclinaison du poinçon de compression incliné est de 25° d'arc.

10. Procédé selon la revendication 6, dans lequel le comprimé obtenu a un côté périphérique en forme de capsule ou de tige.

11. Poinçon de compression utilisé dans l'une quelconque des revendications 6 à 10, caractérisé en ce qu'il a une surface de compression inclinée.

12. Poinçon de compression selon la revendication 11 dont l'angle d'inclinaison de la face de compression inclinée est de 5 à 25° d'arc.

13. Utilisation d'un comprimé comprenant des faces comprimées dont au moins une est inclinée et qui est caractérisé par un gradient de compression qui s'accroît vers l'extrémité plus mince du comprimé, pour la libération contrôlée de l'ingrédient actif.

14. Utilisation selon la revendication 13 où l'angle d'inclinaison des faces inclinées est de 5 à 25° d'arc.

15. Utilisation selon la revendication 13 où l'angle d'inclinaison des faces inclinées est de 25° d'arc.

16. Utilisation selon la revendication 13 où l'angle d'inclinaison des faces inclinées est de 10° d'arc.

17. Utilisation selon la revendication 13 où le côté périphérique du comprimé est en forme de capsule ou de tige.

**FIG 1a**

**FIG 1b**

FIG 2

**FIG 3**

**FIG 4**